# EUROPEAN PATENT SPECIFICATION

(11) **EP 0 629 395 B1**
(45) Date of publication and mention of the grant of the patent: **01.10.1997**
(21) Application number: 94106684.7
(22) Date of filing: 28.04.1994
(51) Int. Cl.: A61K 7/09, A61K 7/06

(54) **Keratinous fiber treatment compositions**
Zusammensetzungen zur Behandlung von Keratinfasern
Composés pour le traitement des fibres kératineuses

(30) Priority: 28.04.1993 JP 102956/93
(43) Date of publication of application: 21.12.1994
(73) Proprietor: Kao Corporation, Chuo-Ku Tokyo 103 (JP)
(72) Inventor: Nagase, Shinobu, Haga-gun, Tochigi (JP); Okada, Tomoyuki, Funabashi-shi, Chiba (JP); Ueda, Shinya, Haga-gun, Tochigi (JP); Sato, Naoki, Haga-gun, Tochigi (JP); Itoh, Takashi, Ichikawa-shi, Chiba (JP)
(74) Representative: Hansen, Bernd, Dr. Dipl.-Chem.

(56) References cited:
- EP-A- 0 455 457
- EP-A- 0 529 437

## Description

### BACKGROUND OF THE INVENTION

### Field of the Invention:

The present invention relates to keratinous fiber treatment compositions with which keratinous fibers such as hair, wool, and fibers for woolen fabrics are chemically treated. More particularly, the present invention relates to keratinous fiber treatment compositions which give keratinous fibers bounce (firmness and resilience), and which enhance the shape-giving effect of curling, waving, straight-perming or the like on keratinous fibers, and which result in the treated keratinous fibers holding the shape for a prolonged period of time. The present invention also relates to a method of treating keratinous fibers with such a composition and kits containing such keratinous fiber treatment compositions.

### Description of Related Art:

Numbers of keratinous fiber treatment compositions have been reported which incorporate polymers and conditioning agents for imparting firmness and resilience to keratinous fibers including hair, wool, and fibers for woolen fabrics (Japanese Patent Application Laid-open (kokai) 7449/1977, Japanese Patent Application Laid-open (kokai) 216113/1983). These compositions exhibit their effects when the compositions adsorb onto the surface of keratinous fibers. However, they result in a poor feeling to the touch, such as a dry and loose feeling, and also cause annoyance by requiring frequent treatments, because the adsorbed treatment compositions come off when the treated fibers are washed.

Moreover, in order to give a new shape such as curls and waves to keratinous fibers, methods including setting with temporary setting agents such as blowing agents for the hair and chemically and semi-permanently treating the hair with perm lotions or the like are typically employed. Keratinous fibers which are set with temporary setting agents lose the shape given by the setting agents when they are exposed to elevated humidity or get wet. On the other hand, curls and waves formed by perming are softened when washed repeatedly, and therefore, the effect of holding the desired shape for a prolonged period is not always satisfactory. Moreover, straight-perming, which reforms frizzy fibers into a straight form, is problematic in that the frizzy keratinous fibers are not always be straightened to the extent desired.

In order to solve these problems, attempts of reforming the fibers themselves in a sustained manner by having treatment agents penetrated into the keratinous fibers have been proposed, which include treatment compositions containing astringents (Japanese Patent Application Nos. 108812/1980, 109405/1983 and 87208/1985), treatment compositions containing naphthalene sulfonic acids (J. Soc. Cosmet. Chem., 36, 87-99 (1985)), treatment compositions which form slightly soluble salts inside the hair fibers (Japanese Patent Application Laid-open (kokai) No.233208/1989) and treatment compositions which contain metallic salts of divalent or higher valence (Japanese Patent Application Laid-open (kokai) No. 275614/1993). Any of these, however, have not produced completely satisfactory results.

Thus, there remains a need for keratinous fiber treatment compositions, which are free of the above-discussed drawbacks, and kits which contain such compositions. There also remains a need for a method of treating keratinous fibers which is effective for giving the fiber a new shape and/or improving the softness, elasticity, and/or resilience of the fibers.

### SUMMARY OF THE INVENTION

Accordingly, it is an object of the present invention to provide novel dual-type keratinous fiber treatment compositions which do not adversely effect the flexible soft touch of the fibers.

It is another object of the present invention to provide keratinous fiber treatment compositions which are effective for giving keratinous fibers bounce (firmness and resilience).

It is another object of the present invention to provide keratinous fiber treatment compositions which are effective for enhancing the shape-giving, i.e, curling, waving or straight-perming, effect of chemical treatments.

It is another object of the present invention to provide keratinous fiber treatment compositions which result in the keratinous fibers holding the shape given to the keratinous fibers for a prolonged period of time.

It is another object of the present invention to provide a method of improving the softness, elasticiy, and/or resilience of keratinous fibers.

It is another object of the present invention to provide novel kits which contain such keratinous fiber treatment compositions.

It is another object of the present invention to provide a method for changing the shape of keratinous fibers.

These and other objects, which will become apparent during the following detailed description, have been achieved by the inventors' discovery that when keratinous fibers are treated with a treatment agent A which contains an aromatic compound having a sulfuric group, a sulfonic group, a phosphoric group, a phosphonic group or a carboxyl group, and with a treatment agent B which contains a polyvalent metal salt in this order or in a reversed order, the elasticity of the keratinous fibers is improved; the flexible soft touch of the fibers is not reduced; a new shape may be given to the fibers, while firmness and resilience is imparted; the shape-giving effect on the fibers effected by chemical treatments such as perming is enhanced; and the newly-given shape lasts for a prolonged period of time.

### DETAILED DESCRIPTION OF THE PREFERRED EMBODIMENTS

According to a first embodiment of the present invention, there is provided a dual-type keratinous fiber treatment composition which is composed of an agent A comprising an aromatic compound having a sulfuric group 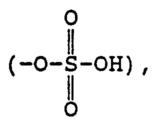 a sulfonic group 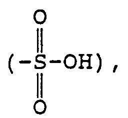 a phosphoric group 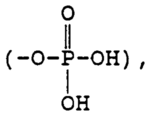 a phosphonic group 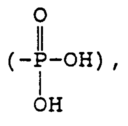 a carboxyl group (-CO₂H), or salts thereof, and an agent B which comprises a polyvalent metal salt.

Among the components of the keratinous fiber treatment compositions of the present invention, preferable aromatic compounds contained in agent A and having a sulfuric group, a sulfonic group, a phosphoric group, a phosphonic group, a carboxyl group, or a salt thereof are those having 1 to 5 aromatic rings of 5 to 7 carbon atoms each. Specific examples of the aromatic rings include a benzene ring, a naphthalene ring, an anthracene ring, an azulene ring, a tetralin ring, an indan ring, a pyrene ring, a fluorene ring and a coumarin ring. Inspection of the list of suitable aromatic rings reveals that the aromatic compound can contain rings which contain saturated carbon atoms, such as in tetralin, indan, and fluorene, and can contain rings which contain heteroatoms and carbonyl groups, in place of a =CH-, such as in coumarin. Of these, the naphthalene ring is particularly preferred. The sulfuric group, sulfonic group, phosphoric group, phosphonic group and the carboxyl group may be present in the aromatic ring-containing compounds singly or in combination of two or more. The sulfuric group, sulfonic group, phosphoric group, phosphonic group, carboxyl group, or salt thereof may be linked to the aromatic ring by a C₁₋₈ alkylene group.

Illustrative examples of the preferable aromatic compounds include those represented by the following formula (1): 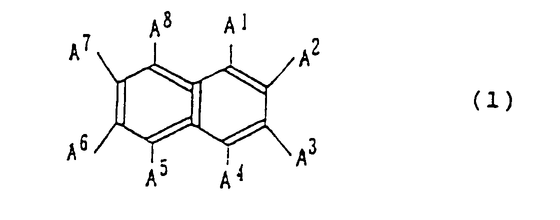 wherein at least one of A¹ to A⁸, which may be the same or different from each other, represents a substituent containing a sulfuric group, a sulfonic group, a phosphoric group, a phosphonic group, a carboxylic group or a salt thereof, and the remainder A¹ to A⁸ represent a hydrogen atom, a halogen atom, a hydroxyl group, a nitro group, an alkyl group having 1 to 10 carbon atoms, an alkenyl group having 2 to 10 carbon atoms, an acyl group having 1 to 10 carbon atoms, an alkoxy group having 1 to 4 carbon atoms, an alkoxycarbonyl group having 2 to 5 carbon atoms, a substituted or unsubstituted phenylazo group, or a group 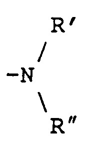 (where R' and R'' may be the same or different and each independently represents a hydrogen atom, a C₁₋₄ alkyl group, a C₁₋₄ alkenyl group, a phenyl group, a benzyl group, or an acyl group having 1 to 10 carbon atoms).

Specific examples of the halogen atom include a fluorine atom, a chlorine atom, a bromine atom and an iodine atom. Specific examples of the alkyl group having 1 to 10 carbon atoms include a methyl group, an ethyl group, an n-propyl group, an isopropyl group, an n-butyl group, a t-butyl group, a pentyl group, a hexyl group, a heptyl group, an octyl group, a nonyl group, and a decyl group. Of these, alkyl groups having 1 to 6 carbon atoms are more preferred. Specific examples of the alkenyl group having 2 to 10 carbon atoms include a vinyl group, an allyl group, a 1-propenyl group, a butenyl group, and a hexenyl group, among which those having 2 to 6 carbon atoms are preferred. Specific examples of the acyl group having 1 to 10 carbon atoms include a formyl group, an acetyl group, a propionyl group, a butyryl group, a pentanoyl group, a hexanoyl group, an octanoyl group, and a decanoyl group, among which alkanoyl groups having 1 to 6 carbon atoms are preferred. Specific examples of the alkoxy group having 1 to 4 carbon atoms include a methoxy group, an ethoxy group, a propoxy group, and a butoxy group. Specific examples of the alkoxycarbonyl group having 2 to 5 carbon atoms include a methoxycarbonyl group, ethoxycarbonyl group, propyloxycarbonyl group and a butyloxycarbonyl group. Specific examples of the substituted or unsubstituted phenylazo group include a phenylazo group, a halogenophenylazo group, a C₁₋₆ alkylphenylazo group and a C₁₋₆ alkoxylphenylazo group. Specific examples of the group 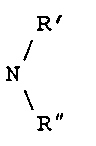 include an amino group, a C₁₋₆ alkylamino group, a di-C₁₋₆ alkylamino group, a C₂₋₆ alkenylamino group, a di-C₂₋₆ alkenylamino group, a phenylamino group, a benzylamino group and a C₁₋₁₀ alkanoylamino group.

Having regard to the A¹ to A⁸, it is preferable that one or two of them are independently a sulfuric group, a sulfonic group, a phosphoric group, a phosphonic group, a carboxyl group or a salt of them, and the remainder individuals are composed of a combination of a hydrogen atom and a member selected from the group consisting of a hydroxyl group, a halogen atom, a nitro group, an alkyl group, an amino group and an imino group. Alternatively, it is also preferable that one or two of A¹ to A⁸ are independently a sulfuric group, a sulfonic group, a phosphoric group, a phosphonic group, a carboxyl group or a salt of them, and one of the remainder individuals are a hydrogen, a hydroxyl group, a halogen atom, a nitro group, an alkyl group, an amino group or an imino group, and the remainder of A¹ to A⁸ are all hydrogen atoms.

Specific examples of the aromatic compounds represented by formula (1) include 1-naphthylsulfuric acid, 2-naphthylsulfuric acid, 1-naphthol-2-sulfuric acid, 2-naphthol-1-sulfuric acid, 2-naphthol-3-sulfuric acid, 1-naphthalenesulfonic acid, 2-naphthalenesulfonic acid, 1-naphthylphosphoric acid, 2-naphthylphosphoric acid, 1-naphthol-2-phosphoric acid, 2-naphthol-1-phosphoric acid, 2-naphthol-3-phosphoric acid, 1-naphthylphosphonic acid, 2-naphthylphosphonic acid, 1-naphthoic acid, 2-naphthoic acid, 1-hydroxy-2-naphthoic acid, 2-hydroxy-1-naphthoic acid, 2-hydroxy-3-naphthoic acid, 2-hydroxy-6-naphthoic acid, 1-naphthylacetic acid, 2-naphthylacetic acid, 2,3-naphthalenedicarboxylic acid, 2,6-naphthalenedicarboxylic acid, 1,2-naphthalenedicarboxylic acid, 1,4-naphthalenedicarboxylic acid, 1,5-naphthalenedicarboxylic acid, 1,8-naphthalenedicarboxylic acid, 8-anilino-1-naphthalenesulfonic acid and their salts.

Besides the above-described aromatic compounds represented by formula (1), other aromatic compounds having a sulfuric group, a sulfonic group, a phosphoric group, a phosphonic group or a carboxyl group may be used. Specific examples of such other aromatic compounds include guaiazulene sulfonic acid, tetralin sulfonic acid, indan sulfonic acid, oxybenzene sulfonic acid, 1-hydroxypyrene-3,6,8-trisulfonic acid, 1,3-dihydroxypyrene-6,8-disulfonic acid, phenylphosphoric acid, fluorescein, carboxyfluorescein, p-phenylbenzoic acid, 7-hydroxycoumarin-3-carboxylic acid, phthalic acid, isophthalic acid, terephthalic acid and their salts.

Among them, particularly preferable ones are 1-naphthalenesulfonic acid, 2-naphthalenesulfonic acid, 1-naphthylphosphoric acid, 2-naphthylphosphoric acid, 1-naphthoic acid, 2-naphthoic acid, 1-naphthylacetic acid, 2-naphthylacetic acid, 1-hydroxy-2-naphthoic acid, 2-hydroxy-1-naphthoic acid, 2-hydroxy-3-naphthoic acid, 2,6-naphthalenedicarboxylic acid, 8-anilino-1-naphthalenesulfonic acid, guaiazulene sulfonic acid, tetralin sulfonic acid, indan sulfonic acid, oxybenzene sulfonic acid, 1-hydroxypyrene-3,6,8-trisulfonic acid, 1,3-dihydroxypyrene-6,8-disulfonic acid, p-phenylbenzoic acid, 7-hydroxycoumarin-3-carboxylic acid, terephthalic acid and their salts.

Examples of the salts of these compounds include sodium salts, potassium salts, lithium salts, ammonium salts, and organic quaternary ammonium salts.

It is preferred that the aromatic compounds having a sulfuric group, sulfonic group, phosphoric group, phosphonic group or a carboxyl group be incorporated into the agent A of the present invention in amounts of 0.1 to 10% by weight, more preferably from 0.5 to 5% by weight (hereinafter simply referred to as %) based on the total weight of the agent A in view of the effect of modifying the hardness of keratinous fibers.

Examples of the polyvalent metal salt contained in the agent B of the keratinous fiber treatment composition according to the present invention include hydroxides; halides such as fluorides, chlorides, bromides and iodides; inorganic acid salts such as sulfates, nitrates, phosphates and carbonates; and organic acid salts such as acetates of metals, such as magnesium, calcium, barium, iron (II), iron (III), manganese, zinc, nickel, aluminum and lanthanum.

Of these polyvalent metal salts, divalent to trivalent inorganic acid salts or divalent to trivalent acetic acid salts are preferred, and particularly, magnesium chloride, magnesium nitrate, magnesium sulfate, magnesium acetate, calcium chloride, calcium nitrate, calcium sulfate, calcium acetate, ferrous chloride, ferrous nitrate, ferrous sulfate, ferrous acetate, ferric chloride, ferric nitrate, ferric sulfate, ferric acetate, zinc chloride, zinc nitrate, zinc sulfate, zinc acetate, aluminum chloride, aluminum nitrate, aluminum sulfate, aluminum acetate, alum and the like are more preferred.

It is preferred that the polyvalent metal salts be incorporated into the agent B of the present invention in amounts of 0.01 to 10% by weight, more preferably from 0.1 to 5% by weight (hereinafter simply referred to as %) based on the total weight of the agent B in view of the effect of modifying the structure of keratinous fibers.

Both agents A and B of the keratinous fiber treatment compositions of the present invention preferably contain an acid or a base as a pH modifier for controlling the pH of the agents in a specific range. When the pH is adjusted to fall within a specific range as described below, keratinous fibers fully swell, thereby allowing the treatment compositions to penetrate inside the fibers even more rapidly. In the case where an acid is used as a pH modifier, the pH of the agents is preferably adjusted to fall in the range of 2 to 5, whereas if a base is used, the pH of the agents is preferably adjusted to fall in the range of 8 to 11. pH values of the agents lower than 2 or higher than 11 may cause damages to keratinous fibers.

Concerning the swelling of keratinous fibers, such as hair in particular, caused by the presence of an acid or a base, there is a report by Bhat et al. {G.R. Bhat et al., J. Soc Cosmet. Chem., 32f, 393-405 (1981)}. In this report, hydrochloric acid was used as the acid, and sodium hydroxide was used as the base. However, in the present invention, it is preferable to use an organic acid or phosphoric acid as the acid, and ammonia or an organic amine as the base.

Examples of the organic acids which serve as the pH modifier according to the present invention include citric acid, glycolic acid, succinic acid, tartaric acid, lactic acid, acetic acid, fumaric acid, malic acid, levulinic acid, butyric acid, valeric acid, oxalic acid, maleic acid, mandelic acid; examples of organic amines include monoethanolamine, diethanolamine, triethanolamine, morpholine, N-methylmorpholine, N-ethylmorpholine, morpholine ethanol, piperidine, piperidine ethanol, aminohydroxymethyl propanediol, 2-amino-2-methyl-1-propanol, 2-amino-2-methyl-1,3-propanediol and basic amino acids such as arginine.

These acids and bases may be used singly or in combination of two or more. They are preferably incorporated into each agent in a proportion ranging from 0.3 to 50%, more preferably from 0.5 to 30% based on the total weight of each agent, in view of the effect of urging keratinous fibers to swell.

It is preferred that each of the agents A and B according to the present invention form a buffer system by the inclusion of sodium salts, potassium salts, ammonium salts or like salts of an organic acid. The same organic acids described above in connection with the pH adjustment can be used as a salt for forming the buffer system.

Each of the agents A and B of the present invention can contain an organic solvent. It is preferred that agent A, in particular, contain an organic solvent so as to increase the solubility of the aromatic compounds having a sulfuric group, a sulfonic group, a phosphoric group, a phosphonic group, or a carboxyl group.

Illustrative examples of the organic solvent include C₁₋₆ alcohols such as ethanol, isopropanol, n-propanol, n-butanol, and isobutanol; diols and triols such as ethylene glycol, propylene glycol, 1,3-butanediol, and glycerol; aromatic alcohols such as benzyl alcohol, 1-phenoxy-2-propanol, cinnamyl alcohol, phenethyl alcohol, p-anisyl alcohol, p-methylbenzyl alcohol, phenoxy ethanol, and 2-bensyloxy ethanol; C₁₋₆ alkylethers such as methylcarbitol, ethylcarbitol, propylcarbitol, butylcarbitol, triethylene glycol monoethyl ether and triethylene glycol monobutyl ether; and N-alkylpyrrolidones such as N-methylpyrrolidone, N-octylpyrrolidone and N-laurylpyrrolidone. The amount of the organic solvents to be incorporated into each agent is preferably from 0.5 to 50%, more preferably from 2 to 30%, based on the total weight of the agent.

In each agent of the present invention, i.e. the agents containing the aforementioned aromatic compounds having a sulfuric group, a sulfonic group, a phosphoric group, a phosphonic group or a carboxyl group, or the polyvalent metal salts, the organic solvents and the pH modifiers, the balance is generally water. However, if necessary and as long as the effects of the invention are not impeded, various additives may also be incorporated, which include thickeners such as xanthane gum, hydroxyethylcellulose, surfactants, perfumes, pearl-gloss imparting agents, colorants, UV absorbers, antioxidants and preservatives. Further, in order to enhance the feel to the touch of treated keratinous fibers, cationic polymers such as cationized cellulose, and silicone derivatives such as dimethylpolysiloxane and amino-modified silicones may also be incorporated.

In the dual-type keratinous fiber treatment compositions of the present invention, the component agents A and B are preserved separately from each other until they are subjected to the fiber treatment. The methods of separate preservation may include the following two types; one is a method by which the component agents A and B are separately preserved in different containers, and the other is a method by which the component agents A and B are separately preserved in a container partitioned into two compartments.

The agents A and B of the present invention may be formulated into a first perm lotion (reducing agent) of a perming treatment by incorporating a reducing agent such as thioglycolic acid, thiolactic acid, cysteine, acetyl cysteine, glutathione, a salt thereof and thioglycolic amide, or may be formulated into a second perm lotion (oxidizing agent) of a perming treatment by incorporating an oxidizing agent such as hydrogen peroxide, sodium bromate, potassium bromate, sodium perborate or the like. Preferably, agent A is formulated with a reducing agent, while agent B is formulated with an oxidizing agent. Suitably, the first perm lotion comprises 0.1 to 10%, preferably 1 to 7%, based on the total weight of the lotion, of the reducing agent, while the second perm lotion comprises 0.5 to 20%, preferably 1 to 15%, based on the total weight of the lotion, of the oxidizing agent.

In the use of the keratinous fiber treatment agents A and B according to the present invention, the keratinous fibers to be treated may be dry or in a damp state after washing. Keratinous fibers are first soaked in or applied with agent A of the present invention. The fibers are then left in contact with agent A to allow agent A to develop for a certain period of time preferably for 5 to 30 minutes. Subsequently, the keratinous fibers treated with agent A are soaked in or applied with agent B, and allowed to stand as is, or the fibers are rinsed after 5 to 30 minutes of developing. Here, order of the use of the two agents may be reversed. That is, agent B may be used first, and agent A may be used thereafter in the same manner as described above. In this connection, after keratinous fibers are soaked in or applied with agents A or B, and during the period of developing for a predetermined period of time, heat may be applied to raise temperature up to 30 to 60°C. Optionally, agent A (or agent B, if it is applied first) may be rinsed from the fibers with water before agent B (or agent A) is applied to the fibers. Water rinsing may also be used to remove the last-applied agent.

Favorable results are also obtainable by incorporating agents A and B into a two-liquid type perm lotion. Specifically, agent A may be contained in a first perm lotion (reducing agent) and agent B in a second perm lotion (oxidizing agent); or agent A may be contained in a second perm lotion (oxidizing agent) and agent B in a first perm lotion (reducing agent). When the present compositions are used in the curling, waving or perming of keratinous fibers, the fiber shaping device (hair curlers, rollers, hair pins, etc.) are preferably applied to the fibers before either agent A or agent B are applied to the fibers.

Preferably, the keratinous fibers are hair. Most preferably, the keratinous fibers are human hair.

In another embodiment, the present invention provides kits which contain the present compositions. In particular, the present kits comprise a first container means, such as a bottle, tube, or foil or plastic bag, which contains agent A, and a second container means, again such as a bottle, tube, or foil or plastic bag, which contains agent B. The kit may also comprise another container means, such as a cardboard or paper box, which contains the first and second container means. The kit may further comprise written instructions for how to use the present compositions. These instructions may be printed directly on any on any one or more of the container means or may be printed on a separate sheet of paper. The kit may also include end papers and a paper or plastic cap to be placed on the head for the purpose of retaining heat during the fiber treatment process.

The keratinous fiber treatment agents according to the present invention never destroy the flexible and soft feel to the touch of the fibers, can give fibers a new shape while imparting firmness and resilience, enhance the shapegiving effect achieved by curling, waving, or perming of keratinous fibers, and help the keratinous fibers hold the imparted shape for a prolonged period of time.

Other features of the invention will become apparent in the course of the following descriptions of exemplary embodiments which are given for illustration of the invention and are not intended to be limiting thereof.

### EXAMPLES

### Examples 1 and 2 and Comparative Examples 1 to 3:

The keratinous fiber treatment compositions shown in Table 1 were prepared, and the effects of imparting firmness and resilience to keratinous fibers were investigated in the following manner. Virgin hair of Japanese women, which had not undergone aesthetic treatments such as cold-perming or bleaching prior to the test, was used as the keratinous fibers. The hair fibers were soaked in the treatment agent A shown in Table 1 for 30 minutes at 40°C, washed with ion-exchange water, and then, soaked in the treatment agent B shown in Table 1 for 15 minutes at 40°C, washed with ion-exchange water and dried. The tensile elasticity was measured under 50% relative humidity. The data were converted into the relative elasticity with respect to the tensile elasticity of untreated hair fibers measured under the same conditions in advance, which was asssigned a value of 1.00. The results are shown in Table 1.

**Table 1**

| Composition (%) | Example 1 | Example 2 | Comparative Example 1 | Comparative Example 2 | Comparative Example 3 |
|---|---|---|---|---|---|
| **Treatment Agent A** | | | | | |
| Benzyl alcohol | 5.0% | 5.0% | 5.0% | 5.0% | 5.0% |
| Ethanol | 20.0 | 20.0 | 20.0 | 20.0 | 20.0 |
| Sodium lactate | 1.2 | 1.2 | 1.2 | 1.2 | 1.2 |
| Lactic acid | 6.0 | 6.0 | 6.0 | 6.0 | 6.0 |
| 1-Naphthylphosphoric acid | 2.0 | - | - | - | - |
| 1-Hydroxy-2-naphthoic acid | - | 2.0 | 2.0 | - | - |
| Water | balance | balance | balance | balance | balance |

| **Treatment Agent B** | | | | | |
|---|---|---|---|---|---|
| Sodium lactate | 1.5 | 1.5 | 1.5 | 1.5 | 1.5 |
| Lactic acid | 6.0 | 6.0 | 6.0 | 6.0 | 6.0 |
| Magnesium chloride | 1.0 | - | - | 1.0 | - |
| Calcium chloride | - | 1.0 | - | - | - |
| Water | balance | balance | balance | balance | balance |
| Tensile elasticity after treatment | 1.32 | 1.35 | 1.10 | 1.08 | 0.98 |

From the tensile elasticity data shown in Table 1, it is understood that the elasticity of keratinous fibers was increased much more by the use of the compositions of Examples 1 and 2 of the present invention than by the use of the compositions of Comparative Examples 1 to 3.

### Examples 3 to 7 and Comparative Example 4:

The keratinous fiber treatment compositions shown in Table 3 were prepared, and the firmness and resilience of keratinous fibers was organoleptically evaluated using, as keratinous fibers, a 10 g tress of virgin hair fibers of Japanese women, which had not undergone aesthetic treatments such as cold-perming or bleaching prior to the test. The tress was treated in the same manner as described in Example 1.

### 1. The evaluation was made by 6 panel members.

The evaluation criteria are shown in Table 2 below.

**Table 2**

| | |
|---|---|
| Significantly improved feel of firmness and resilience compared to untreated hair fibers | A |
| Somewhat improved feel of firmness and resilience compared to untreated hair fibers | B |
| Almost the same level of firmness and resilience as untreated hair fibers | C |

**Table 3**

| Composition (%) | Example 3 | Example 4 | Example 5 | Example 6 | Example 7 | Comparative Example 4 |
|---|---|---|---|---|---|---|
| **Treatment Agent A** | | | | | | |
| Benzyl alcohol | 3.0% | 3.0% | 3.0% | 3.0% | 3.0% | 3.0% |
| Ethanol | 20.0 | 20.0 | 20.0 | 20.0 | 20.0 | 20.0% |
| Sodium citrate | 1.2 | 1.2 | 1.2 | 1.2 | 1.2 | 1.2 |
| Citric acid | 6.0 | 6.0 | 6.0 | 6.0 | 6.0 | 6.0 |
| 2-Naphthylphosphoric acid | 2.0 | 2.0 | - | - | - | - |
| 1-Naphthoic acid | - | - | 2.0 | 2.0 | - | - |
| 2-Hydroxy-3-naphthoic acid | - | - | - | - | 2.0 | - |
| Oxalic acid | - | - | - | - | - | 2.0 |
| Water | balance | balance | balance | balance | balance | balance |

| **Treatment Agent B** | | | | | | |
|---|---|---|---|---|---|---|
| Sodium acetate | 0.5 | 0.5 | 0.5 | 0.5 | 0.5 | 0.5 |
| Acetic acid | 2.0 | 2.0 | 2.0 | 2.0 | 2.0 | 2.0 |
| Calcium chloride | 1.0 | - | 1.0 | - | 1.0 | 1.0 |
| Magnesium chloride | - | 1.0 | - | 1.0 | - | - |
| Water | balance | balance | balance | balance | balance | balance |
| Evaluation of firmness and resilience | A | A | A | A | A | B |

From the results shown in Table 3, it is understood that the firmness and resilience of keratinous fibers were significantly increased by the use of the compositions of Examples 3 to 7 of the present invention when compared to the untreated hair fibers. Moreover, the firmness and resilience obtained by the use of the compositions of the present invention was superior to the result obtained by the use of the composition of Comparative Example 4.

### Examples 8 to 12:

The keratinous fiber treatment compositions shown in Table 4 were prepared. The procedure of Example 3 was followed to investigate the effect of the keratinous fiber treatment compositions of the present invention.

**Table 4**

| Composition (%) | Example 8 | Example 9 | Example 10 | Example 11 | Example 12 |
|---|---|---|---|---|---|
| **Treatment Agent A** | | | | | |
| Benzyl alcohol | 3.0% | 3.0% | 3.0% | 3.0% | 3.0% |
| Ethanol | 10.0 | 10.0 | 10.0 | 10.0 | 10.0 |
| Sodium citrate | 1.2 | 1.2 | 1.2 | 1.2 | 1.2 |
| Citric acid | 6.0 | 6.0 | 6.0 | 6.0 | 6.0 |
| 2-Naphthylacetic acid | 2.0 | 2.0 | - | - | - |
| 8-Anilino-1-naphthalene sulfonic acid | - | - | 2.0 | 2.0 | - |
| 2,6-Naphthalene-dicarboxylic acid | - | - | - | - | 2.0 |
| Water | balance | balance | balance | balance | balance |

| **Treatment Agent B** | | | | | |
|---|---|---|---|---|---|
| Sodium acetate | 0.5 | 0.5 | 0.5 | 0.5 | 0.5 |
| Acetic acid | 2.0 | 2.0 | 2.0 | 2.0 | 2.0 |
| Zinc chloride | 1.0 | - | 1.0 | - | 1.0 |
| Aluminum chloride | - | 1.0 | - | 1.0 | - |
| Water | balance | balance | balance | balance | balance |

The firmness and resilience of keratinous fibers were significantly increased by the use of compositions of Examples 8 to 12 of the present invention.

### Examples 13 to 17:

The keratinous fiber treatment compositions shown in Table 5 were prepared. The procedure of Example 3 was followed to investigate the effect of the keratinous fiber treatment compositions of the present invention.

**Table 5**

| Composition (%) | Example 13 | Example 14 | Example 15 | Example 16 | Example 17 |
|---|---|---|---|---|---|
| **Treatment Agent A** | | | | | |
| Benzyl alcohol | 3.0% | 3.0% | 3.0% | 3.0% | 3.0% |
| Ethanol | 10.0 | 10.0 | 10.0 | 10.0 | 10.0 |
| Sodium citrate | 1.2 | 1.2 | 1.2 | 1.2 | 1.2 |
| Citric acid | 6.0 | 6.0 | 6.0 | 6.0 | 6.0 |
| Guaiazulene sulfonic acid | 2.0 | - | - | - | - |
| Tetralin sulfonic acid | - | 2.0 | - | - | - |
| Indane sulfonic acid | - | - | 2.0 | - | - |
| Oxybenzene sulfonic acid | - | - | - | 2.0 | - |
| p-Phenyl benzoic acid | - | - | - | - | 2.0 |
| Water | balance | balance | balance | balance | balance |

| **Treatment Agent B** | | | | | |
|---|---|---|---|---|---|
| Sodium acetate | 0.5 | 0.5 | 0.5 | 0.5 | 0.5 |
| Acetic acid | 2.0 | 2.0 | 2.0 | 2.0 | 2.0 |
| Calcium chloride | 1.0 | 1.0 | 1.0 | 1.0 | 1.0 |
| Water | balance | balance | balance | balance | balance |

The firmness and resilience of keratinous fibers were significantly increased by the use of compositions of Examples 13 to 17 of the present invention.

### Examples 18 to 22:

The keratinous fiber treatment compositions shown in Table 6 were prepared. The order of the use of treatment agents A and B in Example 1 was changed, and the effect of reforming and improving the structure of keratinous fibers was investigated by organoleptically evaluating the feel of firmness and resilience. As keratinous fibers, a 10 g tress of virgin hair fibers of Japanese women, which had not undergone aesthetic treatments such as cold-perming or bleaching prior to the test, was used. The tress was soaked in treatment agent B shown in Table 6 for 15 minutes at 40°C, washed with ion-exchange water and then, soaked in the treatment agent A shown in Table 6 for 30 minutes at 40°C, washed with ion-exchange water and dried. Organoleptic evaluation in terms of firmness and resilience was carried out in a similar manner as described in Example 3 using the criteria in Table 2. The results are shown in Table 6.

**Table 6**

| Composition (%) | Example 18 | Example 19 | Example 20 | Example 21 | Example 22 |
|---|---|---|---|---|---|
| **Treatment Agent A** | | | | | |
| n-Propanol | 6.0% | 6.0% | 6.0% | 6.0% | 6.0% |
| Ethanol | 15.0 | 15.0 | 15.0 | 15.0 | 15.0 |
| Sodium citrate | 1.2 | 1.2 | 1.2 | 1.2 | 1.2 |
| Citric acid | 6.0 | 6.0 | 6.0 | 6.0 | 6.0 |
| 1-Naphthylphosphoric acid | 2.0 | 2.0 | - | - | - |
| 7-Hydroxycumarin-3-carboxylic acid | - | - | 2.0 | 2.0 | - |
| Terephthalic acid | - | - | - | - | 2.0 |
| Water | balance | balance | balance | balance | balance |

| **Treatment Agent B** | | | | | |
|---|---|---|---|---|---|
| Sodium acetate | 0.5 | 0.5 | 0.5 | 0.5 | 0.5 |
| Glycolic acid | 2.5 | 2.5 | 2.5 | 2.5 | 2.5 |
| Calcium chloride | 1.0 | - | 1.0 | - | 1.0 |
| Zinc chloride | - | 1.0 | - | 1.0 | - |
| Water | balance | balance | balance | balance | balance |
| Evaluation of firmness and resilience | A | A | A | A | A |

The firmness and resilience of keratinous fibers were significantly increased by the use of compositions of Examples 18 to 22 of the present invention.

### Examples 23 to 26 and Comparative Example 5:

The keratinous fiber treatment compositions shown in Table 7 were prepared. The effects of imparting firmness and resilience to keratinous fibers, giving shape to the fibers, and holding the imparted shape were investigated in the following manner. Having regard to the effect of imparting firmness and resilience to keratinous fibers, virgin hair fibers of Japanese men, which had not undergone any aesthetic treatments such as cold-perming or bleaching prior to the test, were used. The hair fibers were soaked in treatment agent A shown in Table 7 for 30 minutes at 40°C, washed with ion-exchange water, and then, soaked in the treatment agent B shown in Table 7 for 15 minutes at 40°C, washed with ion-exchange water and dried. The tensile elasticity was measured under 50% relative humidity. The data were converted into the relative elasticity with respect to the tensile elasticity of untreated hair fibers measured under the same conditions in advance, which was assigned a value of 1.00. Thereafter, the hair fibers were shampooed 20 times, dried again under 50% relative humidity, and the tensile elasticity of the hair fibers was measured. The tensile elasticity data obtained before and after shampooing were compared and the effect of imparting firmness and resilience and holding the same were investigated.

Having regard to the effect of giving shape to the keratinous fibers and holding the imparted shape, virgin hair fibers of Japanese women, which were straight and which had not undergone any aesthetic treatments such as cold perming or bleaching prior to the test, were used. The hair fibers were wound on a rod made of Teflon and having a diameter of 1 cm, and treated with treatment agents A and B as described above. Subsequently, the hair fibers were removed from the rod, dried under 50% relative humidity, and the diameter of the curl formed was measured. Thereafter, the hair fibers were shampooed 20 times, dried again under 50% humidity, and the diameter of the curl was measured. The data obtained before and after shampooing were compared to determine the effects of the compositions with regard to the shape-giving property and the property of holding the shape formed.

**Table 7**

| Composition (%) | Example 23 | Example 24 | Example 25 | Example 26 | Comparative Example 5 |
|---|---|---|---|---|---|
| **Treatment Agent A** | | | | | |
| Benzyl alcohol | 3.0% | 3.0% | 3.0% | 3.0% | 3.0% |
| Ethanol | 10.0 | 10.0 | 10.0 | 10.0 | 10.0 |
| Sodium tartrate | 2.4 | 2.4 | 2.4 | 2.4 | 2.4 |
| Tartaric acid | 12.0 | 12.0 | 12.0 | 12.0 | 12.0 |
| 1-Naphthylphosphoric acid | 3.0 | 3.0 | - | - | 3.0 |
| 2-Naphthylphosphoric acid | - | - | 3.0 | - | - |
| 1-Hydroxypyrene-3,6,8-trisulfonic acid | - | - | - | 3.0 | - |
| Water | balance | balance | balance | balance | balance |

| **Treatment Agent B** | | | | | |
|---|---|---|---|---|---|
| Sodium lactate | 1.5 | 1.5 | 1.5 | 1.5 | 1.5 |
| Lactic acid | 6.0 | 6.0 | 6.0 | 6.0 | 6.0 |
| Ferrous chloride | 1.5 | - | 1.5 | - | - |
| Calcium sulfate | - | 1.5 | - | 1.5 | - |
| Water | balance | balance | balance | balance | balance |

The keratinous fibers treated with the keratinous fiber treatment compositions of Examples 23 to 26 according to the present invention exhibited high tensile elasticity compared to those treated with the composition of Comparative Example 5 when measured immediately after treatment. In addition, the change in tensile elasticity of the hair fibers treated with the compositions of the present invention after repeated shampooing was small. Consequently, it was determined that the keratinous fiber treatment compositions of the present invention impart firmness and resilience to the fibers, and the imparted properties withstand repeated shampooing. With regard to the shape-giving effect on keratinous fibers, the compositions in Examples 23 to 26 according to the present invention provided curls with small diameters, which indicated effective shape-giving performance, when measured immediately after treating the fibers as compared to the compositions of Comparative Example 5. Moreover, the change in the curl diameter after repeated shampooing was small, which apparently indicated the lasting power of shape-giving property of the compositions of the present invention on keratinous fibers.

### Examples 27 to 30 and Comparative Example 6:

The treatment agents A (1st perm lotion, which is a reducing agent) and treatment agents B (2nd perm lotion, which is an oxidizing agent) shown in Table 8 were prepared, and the effect of the inventive compositions on treating hair fibers was investigated in the following manner. As keratinous fibers, virgin hair fibers of Japanese women, which had not undergone any aesthetic treatments such as cold-perming or bleaching prior to the test, were used. The fibers were subjected to a so-called two-liquid type perming treatment. In detail, they were first soaked in treatment agent A (1st perm lotion, which is a reducing agent) shown in Table 8 at 30°C for 10 minutes and then soaked in treatment agent B (2nd perm lotion, which is an oxidising agent, 8% aqueous solution of sodium bromate) shown in Table 8 at 30°C for 10 minutes. The thus treated fibers were washed with ion-exchange water and dried. Tensile elasticity was measured under 50% relative humidity. The data were converted into relative tensile elasticity with respect to the tensile elasticity of untreated hair fibers measured under the same conditions in advance, which was assigned a value of 1.00. Thereafter, the hair fibers were shampooed 20 times, dried again under 50% humidity, and the tensile elasticity of the fibers were measured. The data obtained before and after shampooing were compared to determine the properties of the compositions with regard to the performance of imparting firmness and resilience and its sustainability.

**Table 8**

| Composition (%) | Example 27 | Example 28 | Example 29 | Example 30 | Comparative Example 6 |
|---|---|---|---|---|---|
| **Treatment Agent A** | | | | | |
| Thioglycolic acid | 6.0% | 6.0% | 6.0% | 6.0% | 6.0% |
| Ammonium hydrogen carbonate | 3.0 | 3.0 | 3.0 | 3.0 | 3.0 |
| Ammonia | 0.3 | 0.3 | 0.3 | 0.3 | 0.3 |
| EDTA-2Na | 0.5 | 0.5 | 0.5 | 0.5 | 0.5 |
| Benzyl alcohol | 3.0 | 3.0 | 3.0 | 3.0 | 3.0 |
| Ethanol | 10.0 | 10.0 | 10.0 | 10.0 | 10.0 |
| Sodium 2-naphthalenesulfonate | 3.0 | 3.0 | - | - | 3.0 |
| 1,3-Dihydroxypyrene-6,8-disulfonic acid | - | - | 3.0 | - | - |
| Sodium 2-hydroxy-3-naphthoate | - | - | - | 3.0 | - |
| Water | balance | balance | balance | balance | balance |

| **Treatment Agent B** | | | | | |
|---|---|---|---|---|---|
| Sodium bromate | 8.0 | 8.0 | 8.0 | 8.0 | 8.0 |
| Sodium acetate | 0.5 | 0.5 | 0.5 | 0.5 | 0.5 |
| Acetic acid | 2.0 | 2.0 | 2.0 | 2.0 | 2.0 |
| Alum | 1.0 | - | 1.0 | - | - |
| Magnesium sulfate | - | 1.0 | - | 1.0 | - |
| Water | balance | balance | balance | balance | balance |

The keratinous fibers treated with the keratinous fiber treatment compositions of Examples 27 to 30 according to the present invention exhibited high tensile elasticity as compared to those treated with the composition of Comparative Example 6 when measured immediately after treatment. In addition, the change in tensile elasticity of the hair fibers treated with the compositions of the present invention was small after repeated shampooing. Consequently, it was determined that the keratinous fiber treatment compositions of the invention, when formulated into perm lotions, impart firmness and resilience to the fibers, eliminate damage to the fibers by chemical treatment, and the imparted properties withstand repeated shampooing. With regard to the shape-giving effect to keratinous fibers when the keratinous fiber treatment compositions were formulated into perm lotions, the compositions in Examples 27 to 30 according to the present invention provided curls with small diameters, which indicated effective shape-giving performance, when measured immediately after treating the fibers as compared to the composition of Comparative Example 6. Moreover, changes in the curl diameter after repeated shampooing were small, which apparently indicated the lasting power of the shape-giving property of the inventive compositions on keratinous fibers.

The present application is based on Japense Patent Application No. 102956/1993, filed on April 28, 1993, which is incorporated herein by reference.

Obviously, numerous modifications and variations of the present invention are possible in light of the above teachings. It is therefore to be understood that, within the scope of the appended claims, the invention may be practiced otherwise than as specifically described herein.

## Claims

1. A dual-type keratinous fiber treatment composition which comprises treatment agents A and B; said agent A comprising an aromatic compound having a sulfuric group, a sulfonic group, a phosphoric group, a phosphonic group, a carboxyl group, or a salt thereof, and said agent B comprising a polyvalent metal salt.

2. The composition according to Claim 1, wherein said aromatic compound has 1 to 5 aromatic rings having 5 to 7 carbon atoms in each ring.

3. The composition according to Claim 1, wherein said aromatic compound has an aromatic ring selected from the group consisting of benzene, naphthalene, anthracene, azulene, tetralin, indan, pyrene, fluorene and coumarin.

4. The composition according to Claim 1, wherein said aromatic compound has a naphthalene ring.

5. The composition according to Claim 1, wherein said aromatic compound is represented by the following formula (1): 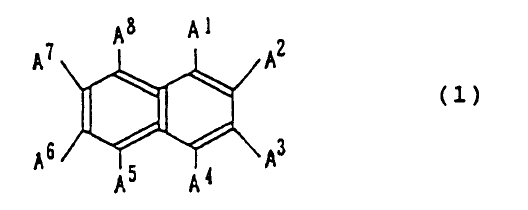 wherein at least one of A¹ to A⁸ , which may be the same or different from each other, represents a substituent containing a sulfuric group, a sulfonic group, a phosphoric group, a phosphonic group, a carboxylic group, or a salt thereof, and the remainder of A¹ to A⁸ represent a hydrogen atom, a halogen atom, a hydroxyl group, a nitro group, an alkyl group having 1 to 10 carbon atoms, an alkenyl group having 2 to 10 carbon atoms, an acyl group having 1 to 10 carbon atoms, an alkoxy group having 1 to 4 carbon atoms, an alkoxycarbonyl group having 2 to 5 carbon atoms, a substituted or unsubstituted phenylazo group,
or a group 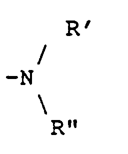 where R' and R'' may be the same or different and each independently represents a hydrogen atom, a C₁₋₄ alkyl group, a C₁₋₄ alkenyl group, a phenyl group, a benzyl group, or an acyl group having 1 to 10 carbon atoms.

6. The composition according to Claim 5, wherein one or two of the A¹ to A⁸ in formula (1) are independently a sulfuric group, a sulfonic group, a phosphoric group, a phosphonic group, a carboxyl group, or a salt thereof; and the remainder of A¹ to A⁸ are selected from the group consisting of hydrogen atom, hydroxy group, halogen atom, nitro group, C₁₋₁₀ alkyl group amino group, and imino group.

7. The composition according to Claim 5, wherein one or two of the A¹ to A⁸ in formula (1) are independently a sulfuric group, a sulfonic group, a phosphoric group, a phosphonic group, a carboxyl group, or a salt thereof; and one of the remainder of A¹ to A⁸ is a hydrogen atom, a hydroxyl group, a halogen atom, a nitro group, a C₁₋₁₀ alkyl group, an amino group or an imino group, and the remainder of A¹ to A⁸ are hydrogen.

8. The composition according to Claim 1, wherein said polyvalent metal salt is a hydroxide, a halide, an inorganic acid salt, or an organic acid salt of a polyvalent metal selected from the group consisting of magnesium, calcium, barium, iron (II), iron (III), manganese, zinc, nickel, aluminum, and lanthanum.

9. The composition according to Claim 1, wherein said aromatic compound is incorporated in said agent A in an amount of 0.1 to 10% by weight, and said polyvalent metal salt is incorporated in said agent B in an amount of 0.01 to 10% by weight, based on the total weight of each agent.

10. The composition according to Claim 1, wherein the pH of said agents A and B is adjusted to fall in the range of 2 to 5 or 8 to 11 by an acid or a base incorporated in the agents.

11. The composition according to Claim 1, wherein said agent A further comprises an organic solvent.

12. The composition according to Claim 1, wherein said agent A further comprises an organic solvent selected from the group consisting of C₁ - C₆ alcohols, diols, triols, aromatic alcohols, polyoxyalkylene C₁ - C₆ alkylethers and N-alkylpyrrolidones.

13. The composition according to Claim 1, wherein either one of said agent A or agent B comprises an oxidizing agent and the other of said agent A or agent B comprises a reducing agent.

14. The composition according to Claim 1, wherein said agent A comprises an organic solvent and a reducing agent, and said agent B comprises an oxidizing agent.

15. A method of treating keratinous fibers, comprising
(i) contacting said keratinous fibers with a first agent to obtain partially treated keratinous fibers; and
(ii) contacting said partially treated keratinous fibers with a second agent; wherein
(a) said first agent is an agent A which comprises an aromatic compound having a sulfuric group, a sulfonic group, a phosphoric group, a phosphonic group, a carboxyl group, or a salt thereof and said second agent is an agent B which comprises a polyvalent metal salt; or
(b) said first agent is an agent B which comprises a polyvalent metal salt and said second agent is an agent A which comprises an aromatic compound having a sulfuric group, a sulfonic group, a phosphoric group, a phosphonic group, a carboxyl group, or a salt thereof.

16. The method of Claim 15, wherein said aromatic compound has 1 to 5 aromatic rings having 5 to 7 carbon atoms in each ring.

17. The method of Claim 15, wherein said aromatic compound has an aromatic ring selected from the group consisting of benzene, naphthalene, anthracene, azulene, tetralin, indan, pyrene, fluorene and coumarin.

18. The method of Claim 15, wherein said aromatic compound has a naphthalene ring.

19. The method of Claim 15, wherein said aromatic compound is represented by the following formula (1): 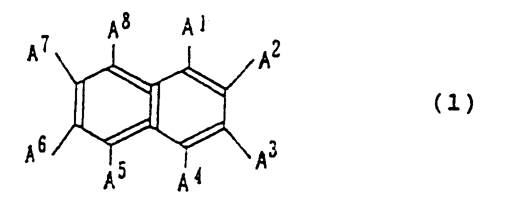 wherein at least one of A¹ to A⁸ , which may be the same or different from each other, represents a substituent containing a sulfuric group, a sulfonic group, a phosphoric group, a phosphonic group, a carboxylic group, or a salt thereof, and the remainder of A¹ to A⁸ represent a hydrogen atom, a halogen atom, a hydroxyl group, a nitro group, an alkyl group having 1 to 10 carbon atoms, an alkenyl group having 2 to 10 carbon atoms, an acyl group having 1 to 10 carbon atoms, an alkoxy group having 1 to 4 carbon atoms, an alkoxycarbonyl group having 2 to 5 carbon atoms, a substituted or unsubstituted phenylazo group,
or a group 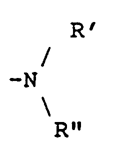 where R' and R'' may be the same or different and each independently represents a hydrogen atom, a C₁₋₄ alkyl group, a C₁₋₄ alkenyl group, a phenyl group, a benzyl group, or an acyl group having 1 to 10 carbon atoms.

20. The method of Claim 19, wherein one or two of the A¹ to A⁸ in formula (1) are independently a sulfuric group, a sulfonic group, a phosphoric group, a phosphonic group, a carboxyl group, or a salt thereof; and the remainder of A¹ to A⁸ are selected from the group consisting of hydrogen atom, hydroxy group, halogen atom, nitro group, C₁₋₁₀ alkyl group, amino group, and imino group.

21. The method of Claim 19, wherein one or two of the A¹ to A⁸ in formula (1) are independently a sulfuric group, a sulfonic group, a phosphoric group, a phosphonic group, a carboxyl group, or a salt thereof; and one of the remainder of A¹ to A⁸ is selected from the group consisting of hydrogen atom, hydroxy group, halogen atom, nitro group, alkyl group, amino group or imino group, and the remainder of A¹ to A⁸ are hydrogen.

22. The method of Claim 15, wherein said polyvalent metal salt is a hydroxide, a halide, an inorganic acid salt, or an organic acid salt of a polyvalent metal selected from the group consisting of magnesium, calcium, barium, iron (II), iron (III), manganese, zinc, nickel, aluminum, and lanthanum.

23. The method of Claim 15, wherein said aromatic compound is incorporated in said agent A in an amount of 0.1 to 10% by weight, and said polyvalent metal salt is incorporated in said agent B in an amount of 0.01 to 10% by weight based on the total weight of each agent.

24. The method of Claim 15, wherein the pH of said agents A and B is adjusted to fall in the range of 2 to 5 or 8 to 11 by an acid or a base incorporated in the agents.

25. The method of Claim 15, wherein said agent A further comprises an organic solvent.

26. The method of Claim 15, wherein said agent A further comprises an organic solvent selected from the group consisting of C₁ - C₆ alcohols, diols, triols, aromatic alcohols, polyoxyalkylene C₁ - C₆ alkylethers and N-alkylpyrrolidones.

27. The method of Claim 15, wherein either one of said agent A or agent B comprises an oxidizing agent and the other of said agent A or agent B comprises a reducing agent.

28. The method of Claim 15, wherein said agent A comprises an organic solvent and a reducing agent, and said agent B comprises an oxidizing agent.

29. A kit for treating keratinous fibers, comprising:
(i) a first container means containing an agent A, which comprises an aromatic compound having a sulfuric group, a sulfonic group, a phosphoric group, a phosphonic group, a carboxyl group, or a salt thereof; and
(ii) a second container means containing an agent B which comprises a polyvalent metal salt.

30. The kit of Claim 29, wherein said aromatic compound has 1 to 5 aromatic rings having 5 to 7 carbon atoms in each ring.

31. The kit of Claim 29, wherein said aromatic compound has an aromatic ring selected from the group consisting of benzene, naphthalene, anthracene, azulene, tetralin, indan, pyrene, fluorene and coumarin.

32. The kit of Claim 29, wherein said aromatic compound has a naphthalene ring.

33. The kit of Claim 29, wherein said aromatic compound is represented by the following formula (1): 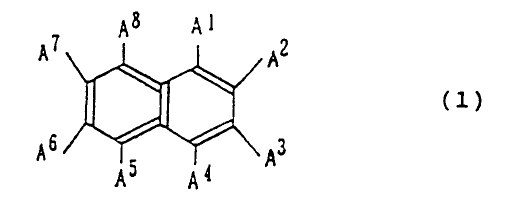 wherein at least one of A¹ to A⁸ , which may be the same or different from each other, represents a substituent containing a sulfuric group, a sulfonic group, a phosphoric group, a phosphonic group, a carboxylic group, or a salt thereof, and the remainder of A¹ to A⁸ represent a hydrogen atom, a halogen atom, a hydroxyl group, a nitro group, an alkyl group having 1 to 10 carbon atoms, an alkenyl group having 2 to 10 carbon atoms, an acyl group having 1 to 10 carbon atoms, an alkoxy group having 1 to 4 carbon atoms, an alkoxycarbonyl group having 2 to 5 carbon atoms, a substituted or unsubstituted phenylazo group,
or a group 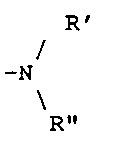 where R' and R'' may be the same or different and each independently represents a hydrogen atom, a C₁₋₄ alkyl group, a C₁₋₄ alkenyl group, a phenyl group, a benzyl group, or an acyl group having 1 to 10 carbon atoms.

34. The kit of Claim 33, wherein one or two of the A¹ to A⁸ in formula (1) are independently a sulfuric group, a sulfonic group, a phosphoric group, a phosphonic group, a carboxyl group, or a salt thereof; and the remainder of A¹ to A⁸ are selected from the group consisting of hydrogen atom, hydroxy group, halogen atom, nitro group, C₁₋₁₀ alkyl group, amino group, and imino group.

35. The kit of Claim 33, wherein one or two of the A¹ to A⁸ in formula (1) are independently a sulfuric group, a sulfonic group, a phosphoric group, a phospbonic group, a carboxyl group, or a salt thereof; and one of the remainder of A¹ to A⁸ is selected from the group consisting of hydrogen, hydroxyl group, halogen atom, nitro group, alkyl group, amino group or imino group, and the remainder of A¹ to A⁸ are hydrogen.

36. The kit of Claim 29, wherein said polyvalent metal salt is a hydroxide, a halide, an inorganic acid salt, or an organic acid salt of a polyvalent metal selected from the group consisting of magnesium, calcium, barium, iron (II), iron (III), manganese, zinc, nickel, aluminum, and lanthanum.

37. The kit of Claim 29, wherein said aromatic compound is incorporated in said agent A in an amount of 0.1 to 10% by weight, and said polyvalent metal salt is incorporated in said agent B in an amount of 0.01 to 10% by weight based on the total weight of each agent.

38. The kit of Claim 29, wherein the pH of said agents A and B is adjusted to fall in the range of 2 to 5 or 8 to 11 by an acid or a base incorporated in the agents.

39. The kit of Claim 29, wherein said agent A further comprises an organic solvent.

40. The kit of Claim 29, wherein said agent A further comprises an organic solvent selected from the group consisting of C₁ - C₆ alcohols, diols, triols, aromatic alcohols, polyoxyalkylene C₁ - C₆ alkylethers and N-alkylpyrrolidones.

41. The kit of Claim 29, wherein either one of said agent A or agent B comprises an oxidizing agent and the other of said agent A or agent B comprises a reducing agent.

42. The kit of Claim 29, wherein said agent A comprises an organic solvent and a reducing agent, and said agent B comprises an oxidizing agent.

## Patentansprüche

1. Keratinfaser-Behandlungszusammensetzung vom Dual-Typ, umfassend Behandlungsmittel A und B; wobei das Mittel A eine aromatische Verbindung mit einer Schwefelsäure-Gruppe, Sulfonsäure-Gruppe, Phosphorsäure-Gruppe, Phosphonsäure-Gruppe, Carboxyl-Gruppe oder einem Salz davon enthält, und das Mittel ein polyvalentes Metallsalz enthält.

2. Zusammensetzung nach Anspruch 1, worin die aromatische Verbindung 1 bis 5 aromatische Ringe mit 5 bis 7 Kohlenstoffatomen in jedem Ring hat.

3. Zusammensetzung nach Anspruch 1, worin die aromatische Verbindung einen aromatischen Ring hat, ausgewählt aus der Gruppe, bestehend aus Benzol, Naphthalin, Anthracen, Azulen, Tetralin, Indan, Pyren, Fluoren und Coumarin.

4. Zusammensetzung nach Anspruch 1, worin die aromatische Verbindung einen Naphthalin-Ring hat.

5. Zusammensetzung nach Anspruch 1, worin die aromatische Verbindung durch die folgende Formel (1) dargestellt ist: 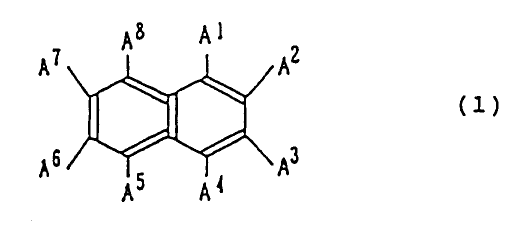 worin zumindest eines von A¹ bis A⁸, die gleich oder verschieden voneinander sein können, einen Substituenten mit einer Schwefelsäure-Gruppe, Sulfonsäure-Gruppe, Phosphorsäure-Gruppe, Phosphonsäure-Gruppe, Carbonsäure-Gruppe oder einem Salz davon darstellt und die verbleibenden von A¹ bis A⁸ ein Wasserstoffatom, Halogenatom, Hydroxyl-Gruppe, Nitro-Gruppe, Alkyl-Gruppe mit 1 bis 10 Kohlenstoffatomen, Alkenyl-Gruppe mit 2 bis 10 Kohlenstoffatomen, Acyl-Gruppe mit 1 bis 10 Kohlenstoffatomen, Alkoxy-Gruppe mit 1 bis 4 Kohlenstoffatomen, Alkoxycarbonyl-Gruppe mit 2 bis 5 Kohlenstoffatomen, substituierte oder unsubstituierte Phenylazo-Gruppe oder eine Gruppe 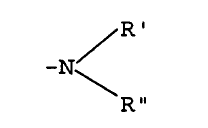 bedeuten, worin R' und R'' gleich oder verschieden voneinander sein können und jeweils unabhängig ein Wasserstoffatom, C₁₋₄-Alkyl-Gruppe, C₁₋₄-Alkenyl-Gruppe, Phenyl-Gruppe, Benzyl-Gruppe oder Acyl-Gruppe mit 1 bis 10 Kohlenstoffatomen sind.

6. Zusammensetzung nach Anspruch 5, worin ein oder zwei von A¹ bis A⁸ in der Formel (1) unabhängig eine Schwefelsäure-Gruppe, Sulfonsäure-Gruppe, Phosphorsäure-Gruppe, Phosphonsäure-Gruppe, Carboxylsäure-Gruppe oder ein Salz davon sind; und die verbleibenden von A¹ bis A⁸ aus der Gruppe ausgewählt sind, bestehend aus Wasserstoffatom, Hydroxy-Gruppe, Halogenatom, Nitro-Gruppe, C₁₋₁₀-Alkyl-Gruppe, Amino-Gruppe und Imino-Gruppe.

7. Zusammensetzung nach Anspruch 5, worin eines oder zwei von A¹ bis A⁸ in der Formel (1) unabhängig eine Schwefelsäure-Gruppe, Sulfonsäure-Gruppe, Phosphorsäure-Gruppe, Phosphonsäure-Gruppe, Carboxyl-Gruppe oder ein Salz davon sind; und eines der verbleibenden von A¹ bis A⁸ ein Wasserstoffatom, Hydroxyl-Gruppe, Halogenatom, Nitro-Gruppe, C₁₋₁₀-Alkyl-Gruppe, Amino-Gruppe oder Imino-Gruppe sind und die verbleibenden von A¹ bis A⁸ Wasserstoffatom sind.

8. Zusammensetzung nach Anspruch 1, worin das polyvalente Metall ein Hydroxid, Halogenid, anorganisches Säuresalz oder organisches Säuresalz eines polyvalenten Metalls ist, ausgewählt aus der Gruppe, bestehend aus Magnesium, Calcium, Barium, Eisen(II), Eisen(III), Mangan, Zink, Nickel, Aluminium und Lanthan.

9. Zusammensetzung nach Anspruch 1, worin die aromatische Verbindung in das Mittel A in einer Menge von 0,1 bis 10 Gew.-% und das polyvalente Metallsalz in das Mittel B in einer Menge von 0,01 bis 10 Gew.-% eingefügt ist, bezogen auf das Gesamtgewicht jeden Mittels.

10. Zusammensetzung nach Anspruch 1, worin der pH der Mittel A und B durch eine Säure oder eine Base, die in die Mittel eingefügt ist, so eingestellt ist, daß er in den Bereich von 2 bis 5 oder 8 bis 11 fällt.

11. Zusammensetzung nach Anspruch 1, worin das Mittel A weiterhin ein organisches Lösungsmittel umfaßt.

12. Zusammensetzung nach Anspruch 1, worin das Mittel A weiterhin ein organisches Lösungsmittel umfaßt, ausgewählt aus der Gruppe, bestehend aus C₁₋₆-Alkoholen, Diolen, Triolen, aromatischen Alkoholen, Polyoxyalkylen-C₁₋₆-alkylethern und N-Alkylpyrrolidonen.

13. Zusammensetzung nach Anspruch 1, worin das Mittel A oder das Mittel B ein Oxidationsmittel und das andere des Mittels A oder B ein Reduktionsmittel umfaßt.

14. Zusammensetzung nach Anspruch 1, worin das Mittel A ein organisches Lösungsmittel und ein Reduktionmittel und das Mittel B ein Oxidationsmittel umfaßt.

15. Verfahren zur Behandlung von Keratinfasern, umfassend
(i) das Kontaktieren der Keratinfasern mit einem ersten Mittel, unter Erhalt von teilweise behandelten Keratinfasern; und
(ii) das Kontaktieren der teilweise behandelten Keratinfasern mit einem zweiten Mittel; worin
(a) das erste Mittel ein Mittel A ist, das eine aromatische Verbindung mit einer Schwefelsäure-Gruppe, Sulfonsäure-Gruppe, Phosphorsäure-Gruppe, Phosphonsäure-Gruppe, Carboxyl-Gruppe oder einem Salz davon umfaßt und das zweite Mittel ein Mittel B ist, das ein polyvalentes Metallsalz umfaßt; oder
(b) das erste Mittel ein Mittel B ist, das ein polyvalentes Metallsalz umfaßt, und das zweite Mittel ein Mittel A ist, das eine aromatische Verbindung mit einer Schwefelsäure-Gruppe, Sulfonsäure-Gruppe, Phosphorsäure-Gruppe, Phosphonsäure-Gruppe, Carboxyl-Gruppe oder einem Salz davon umfaßt.

16. Verfahren nach Anspruch 15, worin die aromatische Verbindung 1 bis 5 aromatische Ringe mit 5 bis 7 Kohlenstoffatomen in jedem Ring hat.

17. Verfahren nach Anspruch 15, worin die aromatische Verbindung einen aromatischen Ring hat, ausgewählt aus der Gruppe, bestehend aus Benzol, Naphthalin, Anthracen, Azulen, Tetralin, Indan, Pyren, Fluoren und Coumarin.

18. Verfahren nach Anspruch 15, worin die aromatische Verbindung einen Naphthalin-Ring hat.

19. Verfahren nach Anspruch 15, worin die aromatische Verbindung durch die folgende Formel (1) dargestellt ist: 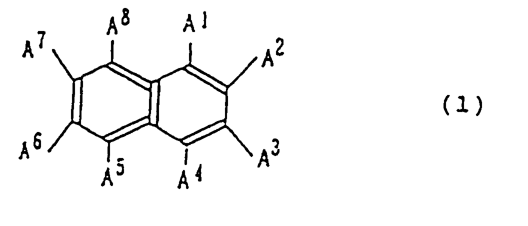 worin zumindest eines von A¹ bis A⁸, die gleich oder verschieden voneinander sein können, einen Substituenten mit einer Schwefelsäure-Gruppe, Sulfonsäure-Gruppe, Phosphorsäure-Gruppe, Phosphonsäure-Gruppe, Carboxyl-Gruppe oder einem Salz davon darstellt und die verbleibenden von A¹ bis A⁸ ein Wasserstoffatom, Halogenatom, Hydroxyl-Gruppe, Nitro-Gruppe, Alkyl-Gruppe mit 1 bis 10 Kohlenstoffatomen, Alkenyl-Gruppe mit 2 bis 10 Kohlenstoffatomen, Acyl-Gruppe mit 1 bis 10 Kohlenstoffatomen, Alkoxy-Gruppe mit 1 bis 4 Kohlenstoffatomen, Alkoxycarbonyl-Gruppe mit 2 bis 5 Kohlenstoffatomen, substituierte oder unsubstituierte Phenylazo-Gruppe oder eine Gruppe 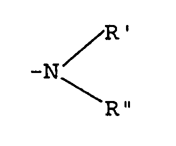 bedeuten, worin R' und R'' gleich oder verschieden voneinander sein können und jeweils unabhängig ein Wasserstoffatom, C₁₋₄-Alkyl-Gruppe, C₁₋₄-Alkenyl-Gruppe, Phenyl-Gruppe, Benzyl-Gruppe oder Acyl-Gruppe mit 1 bis 10 Kohlenstoffatomen sind.

20. Verfahren nach Anspruch 19, worin ein oder zwei von A¹ bis A⁸ in der Formel (1) unabhängig eine Schwefelsäure-Gruppe, Sulfonsäure-Gruppe, Phosphorsäure-Gruppe, Phosphonsäure-Gruppe, Carboxyl-Gruppe oder ein Salz davon sind; und die verbleibenden von A¹ bis A⁸ aus der Gruppe ausgewählt sind, bestehend aus Wasserstoffatom, Hydroxy-Gruppe, Halogenatom, Nitro-Gruppe, C₁₋₁₀-Alkyl-Gruppe, Amino-Gruppe und Imino-Gruppe.

21. Verfahren nach Anspruch 19, worin eines oder zwei von A¹ bis A⁸ in der Formel (1) unabhängig eine Schwefelsäure-Gruppe, Sulfonsäure-Gruppe, Phosphorsäure-Gruppe, Phosphonsäure-Gruppe, Carboxyl-Gruppe oder ein Salz davon sind; und eines der verbleibenden von A¹ bis A⁸ ein Wasserstoffatom, Hydroxyl-Gruppe, Halogenatom, Nitro-Gruppe, Alkyl-Gruppe, Amino-Gruppe oder Imino-Gruppe sind und die verbleibenden von A¹ bis A⁸ Wasserstoffatom sind.

22. Verfahren nach Anspruch 15, worin das polyvalente Metall ein Hydroxid, Halogenid, anorganisches Säuresalz oder organisches Säuresalz eines polyvalenten Metalls ist, ausgewählt aus der Gruppe, bestehend aus Magnesium, Calcium, Barium, Eisen(II), Eisen(III), Mangan, Zink, Nickel, Aluminium und Lanthan.

23. Verfahren nach Anspruch 15, worin die aromatische Verbindung in das Mittel A in einer Menge von 0,1 bis 10 Gew.-% und das polyvalente Metallsalz in das Mittel B in einer Menge von 0,01 bis 10 Gew.-% eingefügt ist, bezogen auf das Gesamtgewicht eines jeden Mittels.

24. Verfahren nach Anspruch 15, worin der pH der Mittel A und B durch eine Säure oder eine Base, die in die Mittel eingefügt ist, so eingestellt ist, daß er in den Bereich von 2 bis 5 oder 8 bis 11 fällt.

25. Verfahren nach Anspruch 15, worin das Mittel A weiterhin ein organisches Lösungsmittel umfaßt.

26. Verfahren nach Anspruch 15, worin das Mittel A weiterhin ein organisches Lösungsmittel umfaßt, ausgewählt aus der Gruppe, bestehend aus C₁₋₆-Alkoholen, Diolen, Triolen, aromatischen Alkoholen, Polyoxyalkylen-C₁₋₆-alkylethern und N-Alkylpyrrolidonen.

27. Verfahren nach Anspruch 15, worin das Mittel A oder das Mittel B ein Oxidationsmittel und das andere des Mittels A oder B ein Reduktionsmittel umfaßt.

28. Verfahren nach Anspruch 15, worin das Mittel A ein organisches Lösungsmittel und ein Reduktionmittel und das Mittel ein Oxidationsmittel umfaßt.

29. Kit zum Behandeln von Keratinfasern, umfassend:
(i) ein erstes Behältermittel, umfassend ein Mittel A, das eine aromatische Verbindung mit einer Schwefelsäure-Gruppe, Sulfonsäure-Gruppe, Phosphorsäure-Gruppe, Phosphonsäure-Gruppe, Carboxyl-Gruppe oder einem Salz davon umfaßt; und
(ii) ein zweites Behältermittel, umfassend ein Mittel B, das ein polyvalentes Metallsalz umfaßt.

30. Kit nach Anspruch 29, worin die aromatische Verbindung 1 bis 5 aromatische Ringe mit 5 bis 7 Kohlenstoffatomen in jedem Ring hat.

31. Kit nach Anspruch 29, worin die aromatische Verbindung einen aromatischen Ring hat, ausgewählt aus der Gruppe, bestehend aus Benzol, Naphthalin, Anthracen, Azulen, Tetralin, Indan, Pyren, Fluoren und Coumarin.

32. Kit nach Anspruch 29, worin die aromatische Verbindung einen Naphthalin-Ring hat.

33. Kit nach Anspruch 29, worin die aromatische Verbindung durch die folgende Formel (1) dargestellt ist: 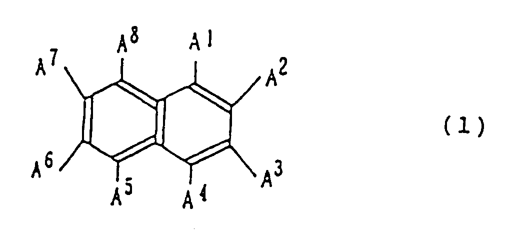 worin zumindest eines von A¹ bis A⁸, die gleich oder verschieden voneinander sein können, einen Substituenten mit einer Schwefelsäure-Gruppe, Sulfonsäure-Gruppe, Phosphorsäure-Gruppe, Phosphonsäure-Gruppe, Carboxyl-Gruppe oder einem Salz davon darstellt und die verbleibenden von A¹ bis A⁸ ein Wasserstoffatom, Halogenatom, Hydroxyl-Gruppe, Nitro-Gruppe, Alkyl-Gruppe mit 1 bis 10 Kohlenstoffatomen, Alkenyl-Gruppe mit 2 bis 10 Kohlenstoffatomen, Acyl-Gruppe mit 1 bis 10 Kohlenstoffatomen, Alkoxy-Gruppe mit 1 bis 4 Kohlenstoffatomen, Alkoxycarbonyl-Gruppe mit 2 bis 5 Kohlenstoffatomen, substituierte oder unsubstituierte Phenylazo-Gruppe oder eine Gruppe 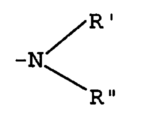 bedeuten, worin R' und R'' gleich oder verschieden voneinander sein können und jeweils unabhängig ein Wasserstoffatom, C₁₋₄-Alkyl-Gruppe, C₁₋₄-Alkenyl-Gruppe, Phenyl-Gruppe, Benzyl-Gruppe oder Acyl-Gruppe mit 1 bis 10 Kohlenstoffatomen sind.

34. Kit nach Anspruch 33, worin ein oder zwei von A¹ bis A⁸ in der Formel (1) unabhängig eine Schwefelsäure-Gruppe, Sulfonsäure-Gruppe, Phosphorsäure-Gruppe, Phosphonsäure-Gruppe, Carboxy-Gruppe oder ein Salz davon sind; und die verbleibenden von A¹ bis A⁸ aus der Gruppe ausgewählt sind, bestehend aus Wasserstoffatom, Hydroxy-Gruppe, Halogenatom, Nitro-Gruppe, C₁₋₁₀-Alkyl-Gruppe, Amino-Gruppe und Imino-Gruppe.

35. Kit nach Anspruch 33, worin eines oder zwei von A¹ bis A⁸ in der Formel (1) unabhängig eine Schwefelsäure-Gruppe, Sulfonsäure-Gruppe, Phosphorsäure-Gruppe, Phosphonsäure-Gruppe, Carboxyl-Gruppe oder ein Salz davon sind; und eines der verbleibenden von A¹ bis A⁸ ein Wasserstoffatom, Hydroxyl-Gruppe, Halogenatom, Nitro-Gruppe, Alkyl-Gruppe, Amino-Gruppe oder Imino-Gruppe sind und die verbleibenden von A¹ bis A⁸ Wasserstoffatom sind.

36. Kit nach Anspruch 29, worin das polyvalente Metall ein Hydroxid, Halogenid, anorganisches Säuresalz oder organisches Säuresalz eines polyvalenten Metalls ist, ausgewählt aus der Gruppe, bestehend aus Magnesium, Calcium, Barium, Eisen(II), Eisen(III), Mangan, Zink, Nickel, Aluminium und Lanthan.

37. Kit nach Anspruch 29, worin die aromatische Verbindung in das Mittel A in einer Menge von 0,1 bis 10 Gew.-% und das polyvalente Metallsalz in das Mittel B in einer Menge von 0,01 bis 10 Gew.-% eingefügt ist, bezogen auf das Gesamtgewicht eines jeden Mittels.

38. Kit nach Anspruch 29, worin der pH der Mittel A und B durch eine Säure oder eine Base, die in die Mittel eingefügt ist, so eingestellt ist, daß er in den Bereich von 2 bis 5 oder 8 bis 11 fällt.

39. Kit nach Anspruch 29, worin das Mittel A weiterhin ein organisches Lösungsmittel umfaßt.

40. Kit nach Anspruch 29, worin das Mittel A weiterhin ein organisches Lösungsmittel umfaßt, ausgewählt aus der Gruppe, bestehend aus C₁₋₆-Alkoholen, Diolen, Triolen, aromatischen Alkoholen, Polyoxyalkylen-C₁₋₆-alkylethern und N-Alkylpyrrolidonen.

41. Kit nach Anspruch 29, worin das Mittel A oder das Mittel B ein Oxidationsmittel und das andere des Mittels A oder B ein Reduktionsmittel umfaßt.

42. Kit nach Anspruch 29, worin das Mittel A ein organisches Lösungsmittel und ein Reduktionmittel und das Mittel B ein Oxidationsmittel umfaßt.

## Revendications

1. Composition de traitement de fibres kératineuses du type double qui comprend des agents de traitement A et B; ledit agent A comportant un composé aromatique ayant un groupe sulfurique, un groupe sulfonique, un groupe phosphorique, un groupe phosphonique, un groupe carboxyle ou un sel de ceux-ci, et ledit agent de traitement B comprenant un sel d'un métal polyvalent.

2. Composition selon la revendication 1, dans laquelle ledit composé aromatique a 1 à 5 cycles aromatiques ayant 5 à 7 atomes de carbone dans chaque cycle.

3. Composition selon la revendication 1, dans laquelle ledit composé aromatique a un cycle aromatique choisi dans le groupe constitué des : benzène, naphtalène, anthracène, azulène, tétraline, indane, pyrène, fluorène et coumarine.

4. Composition selon la revendication 1, dans laquelle ledit composé aromatique a un cycle naphtalène.

5. Composition selon la revendication 1, dans laquelle ledit composé aromatique est représenté par la formule (1) suivante : 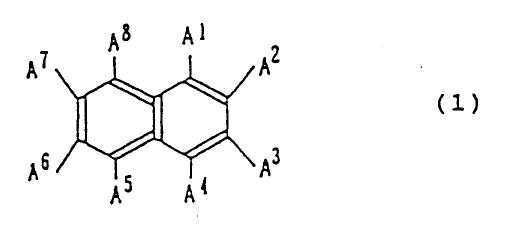 dans laquelle au moins l'un de A¹ à A⁸, qui peuvent être identiques ou différents les uns des autres, représente un substituant contenant un groupe sulfurique, un groupe sulfonique, un groupe phosphorique, un groupe phosphonique, un groupe carboxylique ou un sel de ceux-ci, et le reste des A¹ à A⁸ représente un atome d'hydrogène, un atome d'halogène, un groupe hydroxyle, un groupe nitro, un groupe alkyle ayant 1 à 10 atomes de carbone, un groupe alcényle ayant 2 à 10 atomes de carbone, un groupe acyle ayant 1 à 10 atomes de carbone, un groupe alcoxy ayant 1 à 4 atomes de carbone, un groupe alcoxycarbonyle ayant 2 à 5 atomes de carbone, un groupe phénylazo substitué ou non substitué,
ou un groupe 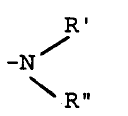 où R' et R'' peuvent être identiques ou différents et chacun représente indépendamment un atome d'hydrogène, un groupe alkyle en C₁-C₄, un groupe alcényle en C₁-C₄, un groupe phényle, un groupe benzyle, ou un groupe acyle ayant 1 à 10 atomes de carbone .

6. Composition selon la revendication 5, dans laquelle un ou deux des A¹ à A⁸ dans la formule (1) sont indépendamment un groupe sulfurique, un groupe sulfonique, un groupe phosphorique, un groupe phosphonique, un groupe carboxyle ou un sel de ceux-ci; et le reste des A¹ à A⁸ est choisi dans le groupe constitué d'un atome d'hydrogène, d'un groupe hydroxy, d'un atome d'halogène, d'un groupe nitro, d'un groupe alkyle en C₁-C₁₀, d'un groupe amino et d'un groupe imino.

7. Composition selon la revendication 5, dans laquelle un ou deux des A¹ à A⁸ dans la formule (1) sont indépendamment un groupe sulfurique, un groupe sulfonique, un groupe phosphorique, un groupe phosphonique, un groupe carboxyle ou un sel de ceux-ci; et l'un du reste des A¹ à A⁸ est un atome d'hydrogène, un groupe hydroxyle, un atome d'halogène, un groupe nitro, un groupe alkyle en C₁-C₁₀, un groupe amino ou un groupe imino, et le reste des A¹ à A⁸ est un atome d'hydrogène.

8. Composition selon la revendication 1, dans laquelle ledit sel de métal polyvalent est un hydroxyde, un halogénure, un sel d'acide inorganique ou un sel d'acide organique d'un métal polyvalent choisi dans le groupe constitué du magnésium, du calcium, du baryum, du fer (II), du fer (III), du manganèse, du zinc, du nickel, de l'aluminium et du lanthane.

9. Composition selon la revendication 1, dans laquelle ledit composé aromatique est incorporé dans ledit agent A dans une quantité de 0,1 à 10 % en poids, et ledit sel de métal polyvalent est incorporé dans ledit agent B dans une quantité de 0,01 à 10 % en poids, par rapport au poids total de chaque agent.

10. Composition selon la revendication 1, dans laquelle le pH desdits agents A et B est ajusté de manière à tomber dans la gamme de 2 à 5 ou de 8 à 11 par un acide ou une base incorporés dans les agents.

11. Composition selon la revendication 1, dans laquelle ledit agent A comprend en outre un solvant organique.

12. Composition selon la revendication 1, dans laquelle ledit agent A comprend en outre un solvant organique choisi dans le groupe constitué des alcools en C₁-C₆, des diols, des triols, des alcools aromatiques, des alkyléthers en C₁-C₆ de polyoxyalkylène et des N-alkylpyrrolidones.

13. Composition selon la revendication 1, dans laquelle l'un ou l'autre dudit agent A ou dudit agent B comprend un agent oxydant et l'autre dudit agent A ou dudit agent B comprend un agent de réduction.

14. Composition selon la revendication 1, dans laquelle ledit agent A comprend un solvant organique et un agent de réduction, et ledit agent B comprend un agent oxydant.

15. Procédé pour traiter des fibres kératineuses, comprenant :
(i) la mise en contact desdites fibres kératineuses avec un premier agent afin d'obtenir des fibres kératineuses partiellement traitées; et
(ii) la mise en contact desdites fibres kératineuses partiellement traitées avec un second agent, dans lequel :
(a) ledit premier agent est un agent A qui comprend un composé aromatique ayant un groupe sulfurique, un groupe sulfonique, un groupe phosphorique, un groupe phosphonique, un groupe carboxyle ou un sel de ceux-ci et ledit second agent est un agent B qui comprend un sel d'un métal polyvalent; ou
(b) ledit premier agent est un agent B qui comprend un sel d'un métal polyvalent et ledit second agent est un agent A qui comprend un composé aromatique ayant un groupe sulfurique, un groupe sulfonique, un groupe phosphorique, un groupe phosphonique, un groupe carboxyle ou un sel de ceux-ci.

16. Procédé selon la revendication 15, dans lequel ledit composé aromatique comporte 1 à 5 cycles aromatiques ayant 5 à 7 atomes de carbone dans chaque cycle.

17. Procédé selon la revendication 15, dans lequel ledit composé aromatique a un cycle aromatique choisi dans le groupe constitué des : benzène, naphtalène, anthracène, azulène, tétraline, indane, pyrène, fluorène et coumarine.

18. Procédé selon la revendication 15, dans lequel ledit composé aromatique a un cycle naphtalène.

19. Procédé selon la revendication 15, dans lequel ledit composé aromatique est représenté par la formule (1) suivante : 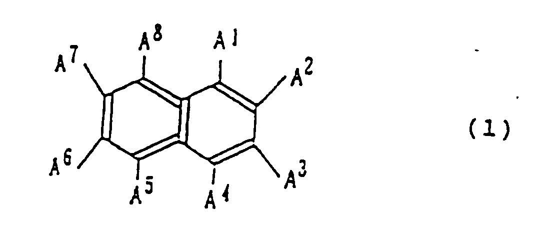 dans laquelle au moins l'un de A¹ à A⁸, qui peuvent être identiques ou différents les uns des autres, représente un substituant contenant un groupe sulfurique, un groupe sulfonique, un groupe phosphorique, un groupe phosphonique, un groupe carboxylique ou un sel de ceux-ci, et le reste des A¹ à A⁸ représente un atome d'hydrogène, un atome d'halogène, un groupe hydroxyle, un groupe nitro, un groupe alkyle ayant 1 à 10 atomes de carbone, un groupe alcényle ayant 2 à 10 atomes de carbone, un groupe acyle ayant 1 à 10 atomes de carbone, un groupe alcoxy ayant 1 à 4 atomes de carbone, un groupe alcoxycarbonyle ayant 2 à 5 atomes de carbone, un groupe phénylazo substitué ou non substitué,
ou un groupe 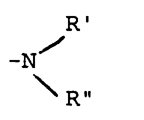 où R' et R'' Peuvent être identiques ou différents et chacun représente indépendamment un atome d'hydrogène, un groupe alkyle en C₁-C₄, un groupe alcényle en C₁-C₄, un groupe phényle, un groupe benzyle, ou un groupe acyle ayant 1 à 10 atomes de carbone.

20. Procédé selon la revendication 19, dans lequel un ou deux des A¹ à A⁸ dans la formule (1) sont indépendamment un groupe sulfurique, un groupe sulfonique, un groupe phosphorique, un groupe phosphonique, un groupe carboxyle ou un sel de ceux-ci; et le reste des A¹ à A⁸ est sélectionné dans le groupe constitué d'un atome d'hydrogène, d'un groupe hydroxy, d'un atome d'halogène, d'un groupe nitro, d'un groupe alkyle en C₁₋₁₀, d'un groupe amino et d'un groupe imino.

21. Procédé selon la revendication 19, dans lequel un ou deux des A¹ à A⁸ dans la formule (1) sont indépendamment un groupe sulfurique, un groupe sulfonique, un groupe phosphorique, un groupe phosphonique, un groupe carboxyle ou un sel de ceux-ci; et l'un du reste des A¹ à A⁸ est choisi dans le groupe constitué d'un atome d'hydrogène, d'un groupe hydroxy, d'un atome d'halogène, d'un groupe nitro, d'un groupe alkyle, d'un groupe amino ou d'un groupe imino, et le reste des A¹ à A⁸ est un atome d'hydrogène.

22. Procédé selon la revendication 15, dans lequel ledit sel de métal polyvalent est un hydroxyde, un halogénure, un sel d'acide inorganique ou un sel d'acide organique d'un métal polyvalent choisi dans le groupe constitué des : magnésium, calcium, baryum, fer (II), fer (III), manganèse, zinc, nickel, aluminium et lanthane.

23. Procédé selon la revendication 15, dans lequel ledit composé aromatique est incorporé dans ledit agent A dans une quantité de 0,1 à 10 % en poids, et ledit sel de métal polyvalent est incorporé dans ledit agent B dans une quantité de 0,01 à 10 % en poids, par rapport au poids total de chaque agent.

24. Procédé selon la revendication 15, dans lequel le pH desdits agents A et B est ajusté de manière à tomber dans la gamme de 2 à 5 ou de 8 à 11 par un acide ou une base incorporés dans les agents.

25. Procédé selon la revendication 15, dans lequel ledit agent A comprend en outre un solvant organique.

26. Procédé selon la revendication 15, dans lequel ledit agent A comprend en outre un solvant organique choisi dans le groupe constitué des alcools en C₁-C₆, des diols, des triols, des alcools aromatiques, des alkyléthers en C₁-C₆ de polyoxyalkylène et des N-alkylpyrrolidones.

27. Procédé selon la revendication 15, dans lequel l'un ou l'autre desdits agent A ou B comprend un agent oxydant et l'autre desdits agent A ou B comprend un agent de réduction.

28. Procédé selon la revendication 15, dans lequel ledit agent A comprend un solvant organique et un agent de réduction, et ledit agent B comprend un agent oxydant.

29. Ensemble pour traiter des fibres kératineuses, comprenant :
(i) un premier moyen de conteneur renfermant un agent A, qui comprend un composé aromatique ayant un groupe sulfurique, un groupe sulfonique, un groupe phosphorique, un groupe phosphonique, un groupe carboxyle ou un sel de ceux-ci; et
(ii) un second moyen de conteneur renfermant un agent B qui comprend un sel d'un métal polyvalent.

30. Ensemble selon la revendication 29, dans lequel ledit composé aromatique a 1 à 5 cycles aromatiques ayant 5 à 7 atomes de carbone dans chaque cycle.

31. Ensemble selon la revendication 29, dans lequel ledit composé aromatique a un cycle aromatique choisi dans le groupe constitué des : benzène, naphtalène, anthracène, azulène, tétraline, indane, pyrène, fluorène et coumarine.

32. Ensemble selon la revendication 29, dans lequel ledit composé aromatique a un cycle naphtalène.

33. Ensemble selon la revendication 29, dans lequel ledit composé aromatique est représenté par la formule (1) suivante : 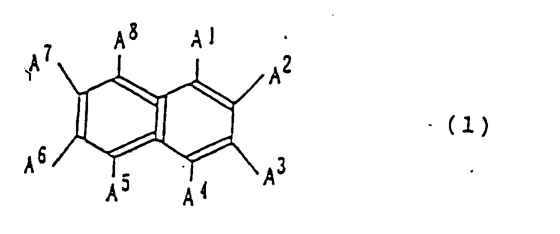 dans laquelle au moins l'un des A¹ à A⁸, qui peuvent être identiques ou différents les uns des autres, représente un substituant contenant un groupe sulfurique, un groupe sulfonique, un groupe phosphorique, un groupe phosphonique, un groupe carboxylique ou un sel de ceux-ci, et le reste des A¹ à A⁸ représente un atome d'hydrogène, un atome d'halogène, un groupe hydroxyle, un groupe nitro, un groupe alkyle ayant 1 à 10 atomes de carbone, un groupe alcényle ayant 2 à 10 atomes de carbone, un groupe acyle ayant 1 à 10 atomes de carbone, un groupe alcoxy ayant 1 à 4 atomes de carbone, un groupe alcoxycarbonyle ayant 2 à 5 atomes de carbone, un groupe phénylazo substitué ou non substitué,
ou un groupe 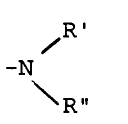 où R' et R'' peuvent être identiques ou différents et chacun représente indépendamment un atome d'hydrogène, un groupe alkyle en C₁-C₄, un groupe alcényle en C₁-C₄, un groupe phényle, un groupe benzyle, ou un groupe acyle ayant 1 à 10 atomes de carbone.

34. Ensemble selon la revendication 33, dans lequel un ou deux des A¹ à A⁸ dans la formule (1) sont indépendamment un groupe sulfurique, un groupe sulfonique, un groupe phosphorique, un groupe phosphonique, un groupe carboxyle ou un sel de ceux-ci; et le reste des A¹ à A⁸ est choisi dans le groupe constitué d'un atome d'hydrogène, d'un groupe hydroxy, d'un atome d'halogène, d'un groupe nitro, d'un groupe alkyle en C₁-C₁₀, d'un groupe amino et d'un groupe imino.

35. Ensemble selon la revendication 33, dans lequel un ou deux des A¹ à A⁸ dans la formule (1) sont indépendamment un groupe sulfurique, un groupe sulfonique, un groupe phosphorique, un groupe phosphonique, un groupe carboxyle ou un sel de ceux-ci; et l'un du reste des A¹ à A⁸ est choisi dans le groupe constitué d'un atome d'hydrogène, d'un groupe hydroxyle, d'un atome d'halogène, d'un groupe nitro, d'un groupe alkyle en C₁₋₁₀, d'un groupe amino ou d'un groupe imino, et le reste des A¹ à A⁸ est un atome d'hydrogène.

36. Ensemble selon la revendication 29, dans lequel ledit sel d'un métal polyvalent est un hydroxyde, un halogénure, un sel d'acide inorganique ou un sel d'acide organique d'un métal polyvalent choisi dans le groupe constitué des : magnésium, calcium, baryum, fer (II), fer (III), manganèse, zinc, nickel, aluminium et lanthane.

37. Ensemble selon la revendication 29, dans lequel ledit composé aromatique est incorporé dans ledit agent A dans une quantité de 0,1 à 10 % en poids, et ledit sel d'un métal polyvalent est incorporé dans ledit agent B dans une quantité de 0,01 à 10 % en poids, par rapport au poids total de chaque agent.

38. Ensemble selon la revendication 29, dans lequel le pH desdits agents A et B est ajusté pour tomber dans la gamme de 2 à 5 ou de 8 à 11 par un acide ou une base incorporés dans les agents.

39. Ensemble selon la revendication 29, dans lequel ledit agent A comprend en outre un solvant organique.

40. Ensemble selon la revendication 29, dans lequel ledit agent A comprend en outre un solvant organique choisi dans le groupe constitué des alcools en C₁-C₆, des diols, des triols, des alcools aromatiques, des alkyléthers en C₁-C₆ de polyoxyalkylène et des N-alkylpyrrolidones.

41. Ensemble selon la revendication 29, dans lequel l'un ou l'autre dudit agent A ou dudit agent B comprend un agent oxydant et l'autre dudit agent A ou dudit agent B comprend un agent de réduction.

42. Ensemble selon la revendication 1, dans lequel ledit agent A comprend un solvant organique et un agent de réduction, et ledit agent B comprend un agent oxydant.
